# EUROPEAN PATENT APPLICATION

(11) **EP 3 766 535 A1**
(43) Date of publication of application: **20.01.2021**
(21) Application number: 20183379.5
(22) Date of filing: 01.07.2020
(51) Int. Cl.: A61M 25/09, A61M 25/01, A61M 25/10

(54) **MOVING A GUIDEWIRE IN A BRAIN LUMEN**

(30) Priority: 02.07.2019 US 201916459803
(71) Applicant: Biosense Webster (Israel) Ltd, Yokneam, 2066717 (IL)
(72) Inventor: GOVARI, Assaf, 2066717 Yokneam (IL); ALGAWI, Yehuda, 2066717 Yokneam (IL)
(74) Representative: Small, Gary James

(57) **Abstract**

A medical probe (28) includes a guidewire (33), a guidewire advancement mechanism (GAM, 55), and a middle inflatable balloon (39). The guidewire (33) is configured for insertion into a lumen of an organ of a patient. The GAM is disposed at a distal end of the guidewire (33), with the GAM including: (i) a proximal inflatable balloon (35), (ii) a distal inflatable balloon (37), and (iii) a middle inflatable balloon (39) that is coupled between the proximal (35) and distal balloons (37). The proximal, distal and middle balloons (35, 37, 39) are configured to move the guidewire (33) in the lumen by inflating and deflating in a predefined sequence.

## Description

### FIELD OF THE INVENTION

The present invention relates generally to invasive medical probes, and particularly to probes for cerebrovascular applications.

### BACKGROUND OF THE INVENTION

Various types of medical probes that include mechanical elements at a distal end of the probe were proposed in the patent literature. For example, U.S. Patent 8,821,476 describes devices and methods for engraftment of stem cells into a pancreas using an endovascular approach. In one embodiment, a catheter device includes expandable occlusion elements in the form of inflatable balloons that can be used to isolate a proximal and distal end of a pancreatic portion of the splenic artery. In another embodiment, the occlusion elements include a filter element instead of a balloon. In some embodiments, an arterial section of the splenic artery can be isolated for selective perfusion of therapeutic cells/drugs to the tail of the pancreas.

As another example, U.S. Patent 5,836,967 describes a catheter assembly for the treatment of vessels carrying body fluid, the catheter comprising an inner catheter provided with a guidewire lumen for receiving a guidewire and an inflation lumen for a balloon provided at its distal end. The catheter assembly further comprises an outer catheter provided with an inflation lumen for a balloon provided at its distal end and a lumen in which the inner catheter is applied, the inner catheter configured to be shifted for changing the spacing of the two balloons. Furthermore, the outer catheter features a larger cross-section than that of the inner catheter so that between the outer wall of the inner catheter and the inner wall of the outer catheter a cross-section employable as an inflation lumen exists and ports into the portion sited between the two balloons.

U.S. Patent 5,632,760 describes a catheter comprising a tube-like basic body having a proximal end, a distal end section, and a distal end. At least one balloon is mounted on a distal end section close to the distal end. The balloon is connected via a lumen in the basic body to the connecting member which is located at the proximal end of the basic tubular body. Two bulges are arranged to the basic body. Each bulge is located close to one end of the balloon. A compressed stent is arranged around the balloon and in between the bulges.

### SUMMARY OF THE INVENTION

An embodiment of the present invention provides a medical probe including a guidewire, a guidewire advancement mechanism (GAM), and a middle inflatable balloon. The guidewire is configured for insertion into a lumen of an organ of a patient. The GAM is disposed at a distal end of the guidewire, with the GAM including: (i) a proximal inflatable balloon, (ii) a distal inflatable balloon, and (iii) a middle inflatable balloon that is coupled between the proximal and distal balloons. The proximal, distal and middle balloons are configured to move the guidewire in the lumen by inflating and deflating in a predefined sequence.

In some embodiments, the medical probe further includes a variable-length element, which is fitted inside the middle balloon and connects the distal balloon and the proximal balloon. In an embodiment, the variable-length element includes a spring.

There is additionally provided, in accordance with an embodiment of the present invention, a system, including a medical probe and an inflation/deflation (I/D) unit. The medical probe includes a guidewire, a guidewire advancement mechanism (GAM), and a middle inflatable balloon. The guidewire is configured for insertion into a lumen of an organ of a patient. The GAM is disposed at a distal end of the guidewire, with the GAM including: (i) a proximal inflatable balloon, (ii) a distal inflatable balloon, and (iii) a middle inflatable balloon that is coupled between the proximal and distal balloons. The proximal, distal and middle balloons are configured to move the guidewire in the lumen by inflating and deflating in a predefined sequence. The inflation/deflation (I/D) unit includes a controller and one or more pumps configured to cause the GAM and the guidewire to move in the lumen, by inflating and deflating the proximal, distal and middle balloons in a predefined sequence.

In some embodiments, the I/D unit is configured to advance the GAM and the guidewire in the lumen by applying the following predefined sequence: (a) inflating the proximal balloon, (b) inflating the middle balloon, (c) inflating the distal balloon, and (d) deflating the proximal balloon and the middle balloon.

In some embodiments, the I/D unit is configured to retract the GAM and the guidewire in the lumen by applying the following predefined sequence: (a) inflating the proximal balloon, (b) inflating the middle balloon, (c) inflating the distal balloon, and (d) deflating the proximal balloon and the middle balloon.

There is additionally provided, in accordance with an embodiment of the present invention, a method, including inserting, into a lumen of an organ of a patient, a guidewire whose distal end is connected to a guidewire advancement mechanism (GAM), the GAM including:(i) a proximal inflatable balloon, (ii) a distal inflatable balloon, and (iii) a middle inflatable balloon that is coupled between the proximal and distal balloons. The GAM and the guidewire are moved in the lumen by inflating and deflating the proximal, distal and middle balloons of the GAM in a predefined sequence.

The present invention will be more fully understood from the following detailed description of the embodiments thereof, taken together with the drawings in which:

### BRIEF DESCRIPTION OF THE DRAWINGS

Figs. 1A and 1B are schematic, pictorial illustrations of cerebrovascular catheter-based position tracking systems, in accordance with embodiments of the present invention;
Fig. 2 is a schematic cross-sectional view of a tortuous section of a brain vessel and of a guidewire advancement mechanism (GAM) configured to transit the tortuous section, in accordance with an embodiment of the present invention; and
Fig. 3 is a flow chart that schematically illustrates a method for advancing a guidewire via a tortuous section of a brain vessel using the GAM of Fig. 2, in accordance with an embodiment of the present invention.

### DETAILED DESCRIPTION OF EMBODIMENTS

### OVERVIEW

Some medical conditions, such as a blockage of a lumen in the body, may require catheterization. In some cases, an urgent catheterization is required for treating an emergency medical condition, such as a stroke or an aneurysm. In addition, chronic cases may also require treatment using an invasive probe, such as with cases of biliary obstruction and in repeated blockage an eustachian tube.

In case of a cerebrovascular condition, such as a clot-induced ischemic stroke or an aneurysm, the location of the clot, or the aneurism, in the brain may be detected by computerized tomography (CT) or fluoroscopy imaging. The affected location, however, may not be readily accessible to a treatment probe, such as a catheter, due to, for example, narrow and/or tortuous sections of brain vessels leading to the affected location. Such narrow and/or tortuous sections may, for example, cause a guidewire of a probe to flex uncontrollably when a physician attempts to advance the guidewire, and thus completely halt distal advancement of the guidewire.

Embodiments of the present invention that are described hereinafter provide systems, probes comprising a guidewire, and methods to enable the guidewire to transit narrow and/or tortuous sections of lumens, such as, but not limited to, brain vessels. The transiting of the guidewire is performed by step-wise advancement of a distal end of the guidewire, as described below.

In some embodiments, a guidewire advancement mechanism (GAM) is disposed at the distal end of the guidewire of the probe (e.g., of a catheter). The GAM comprises a proximal inflatable balloon, a distal inflatable balloon, and a middle inflatable balloon that is coupled between the proximal and distal balloons, wherein the proximal, distal and middle balloons are configured to move the guidewire in the lumen by inflating and deflating in a predefined sequence. Typically, the different balloons are inflated and deflated with saline solution.

In some embodiments, the GAM is configured to distally advance or retract the guidewire in the lumen by the GAM performing a step-wise movement. For the guidewire to move in either direction, the proximal, distal, and middle balloons are inflated and deflated in different predefined sequences. To facilitate the step-wise movement, the middle balloon is capable of alternately extending and retracting when being inflated and deflated, respectively, as described below.

In the context of this disclosure, deflating the middle balloon includes the option to evacuate the middle inflatable balloon to establish sub-pressure therein.

In some embodiments, the probe comprises a variable-length element, which is fitted inside the middle balloon and connects the distal balloon and the proximal balloon. Different implementations of the variable-length element are possible and may include a mechanical element that is either elastic, e.g., a spring, or compressible, such as a telescopic rod that is filled with compressible media, such as gas. In alternative embodiments, the variable-length element is made of a shape-memory alloy, such as Nitinol. The length of a Nitinol-made variable-length element can be changed by changing the temperature of the Nitinol material using, for example, electrical current that is passed via the Nitinol-made element.

In some embodiments, at any given point in a lumen where the guidewire may not be able to pass, a physician operates the GAM to perform the step-wise, "crawling," advancement of the distal end of the guidewire. For that the physician uses a triple-balloon inflation/deflation (I/D) unit, which is configured to sequentially perform these steps:
1. Inflating the proximal balloon of the GAM with saline solution to anchor the balloon against the lumen.
2. Inflating the middle balloon with saline solution to assist the GAM advancement by the elastic/compressible mechanical element extending distally in the process.
3. Inflating the distal balloon of the GAM with saline solution to anchor the GAM against the lumen.
4. Deflating the proximal balloon to become free from the lumen, and evacuating the middle to create sub-pressure inside the balloon, to assist the elastic/compressible element to contract and thereby pull distally the proximal balloon.

Typically, the I/D unit comprises one or more pumps that pump in or pump out the saline solution to perform the inflation and deflation of the balloons.

Using the above described guidewire advancement or guidewire retraction methods, the guidewire effectively "crawls" through any torturous and/or narrow passage in a lumen. The above process is repeated as necessary by the physician operating the probe until the guidewire has advanced to a point where it can be further advanced by being pushed as usual from a handle of the catheter.

In addition to the middle balloon being configured to alternately extend and retract along a longitudinal axis of the guidewire, the middle inflatable balloon element may also provide stiffness in a radial direction to further assist the advancement of the GAM against mechanical resistance inside the lumen.

In some embodiments, the three balloons, and in particular the middle balloon, have particularly low diameters (e.g., up to few milliliters when inflated), so as to further enable advancing the GMA via relatively narrow vessels.

The disclosed GMA-fitted guidewire-guided probes are capable of accessing locations in an organ (e.g., a brain) that require treatment, which may otherwise be inaccessible to a probe. By enabling such access, the disclosed systems, probes, and methods may improve the clinical outcome of medical catheterization procedures.

### SYSTEM DESCRIPTION

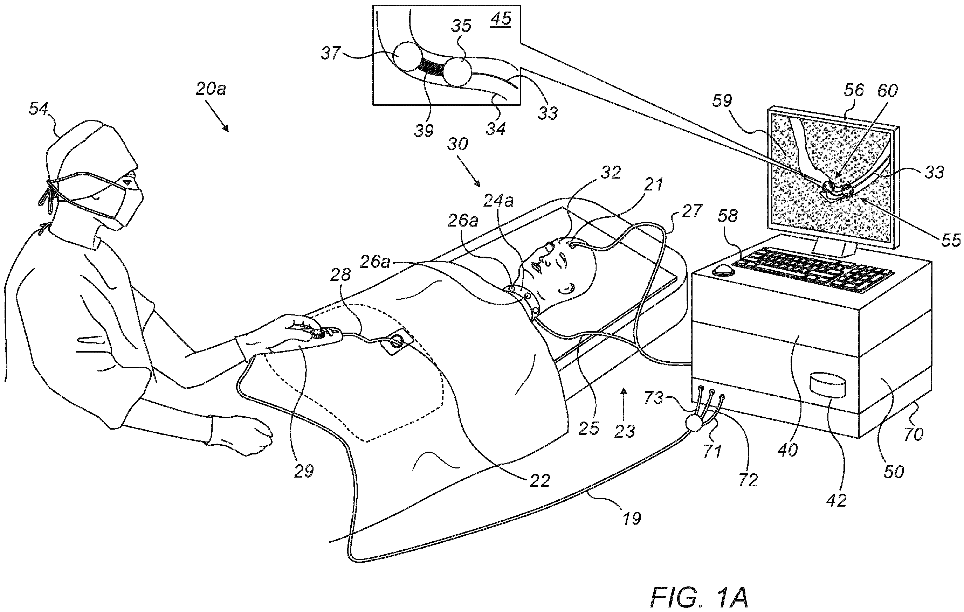

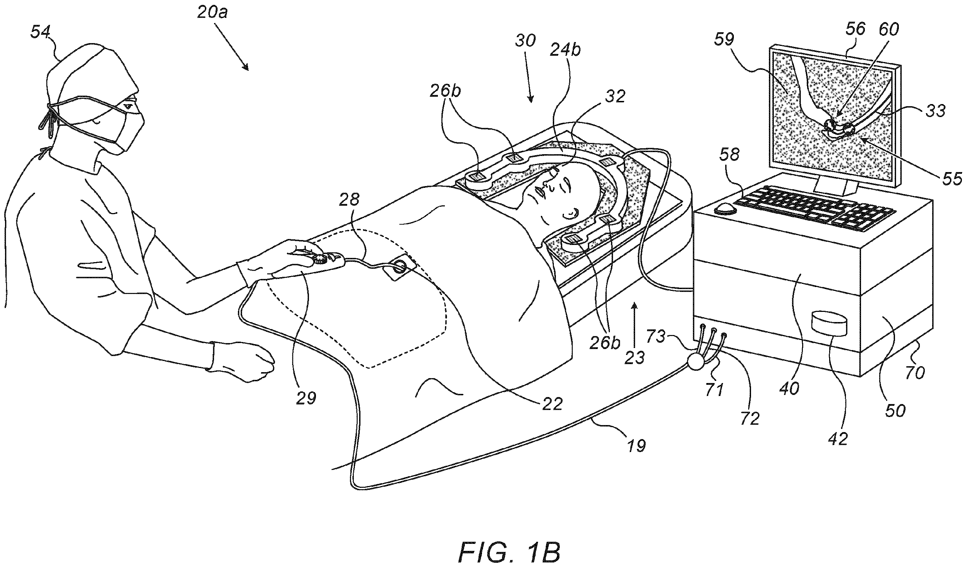

Figs. 1A and 1B are schematic, pictorial illustrations of cerebrovascular catheter-based position tracking systems 20a and 20b, in accordance with embodiments of the present invention.

In some embodiments, prior to performing the catherization procedure, CT images of a patient 22 are acquired. The CT images are stored in a memory 42 for subsequent retrieval by a processor 40. The processor uses the images to present, for example, brain section image 59 demonstrating a clot on a display 56. During the disclosed catheterization procedure, systems 20a and 20b register a position of the distal end of a catheter 28 inside the patient's brain, with frames of reference of brain images of patient 32, herein assumed by way of example to comprise real-time fluoroscopic images. The position of a catheter distal end is tracked using a magnetic tracking sub-system 23, which tracks spatial coordinates of a magnetic sensor fitted at the distal end.

Magnetic tracking sub-system 23 of system 20a, shown in Fig. 1A, comprises a location pad 24a, which is implemented as a collar around the neck of patient 32. By putting location pad 24a around the neck, location pad 24a is configured to automatically compensate for patient head movement. Location pad 24a comprises magnetic field radiators 26a which are fixed in positions relative to the head of patient 32 and which transmit alternating sinusoidal magnetic fields into a region 30 where the head of patient 32 is located. A console 50 electrically drives radiators 26a via a cable 25. In an embodiment, further compensation of head motion is provided by attaching a reference sensor 21 to the patient's forehead. Console 50 is configured to receive signals from reference sensor 21 via a cable 27. A location tracking system that comprises a neck collar location pad is described in U.S. Patent Application 16/248,393, filed January 24, 2019, entitled "Position Sensor on Brain-Clot Sheath and Location Pad Collar," which is assigned to the assignee of the present patent application and whose disclosure is incorporated herein by reference.

Physician 54, operating system 20a, holds catheter controller handle 29, which is connected to the proximal end of catheter 28. Controller 29 allows the physician to advance and navigate catheter 28 in the brain, for example, through an entry point 22 at an artery at a thigh of patient 32. Using magnetic position tracking sub-system 23, a physician 54 advances the distal end of catheter 28 to the clot through blood vessels, usually arteries, so as to enable diagnosis of the type of clot and optionally to perform a corresponding invasive therapeutic procedure to remove the clot. Console 50 receives the position signals from the magnetic position sensor fitted at the distal end of catheter 28 via a cable 19 that connect to catheter 28 via handle 29.

Elements of system 20a, including radiators 26a, are controlled by a system processor 40, comprising a processing unit communicating with one or more memories. Processor 40 may be mounted in console 50, which comprises operating controls 58 that typically include a keypad and/or a pointing device such as a mouse or trackball. Physician 54 uses operating controls on handle 29 to interact with the processor while performing the registration of system 20a. During the registration process, an image 59 of a brain section is presented on display 56. Subsequent to the registration process described above, physician 54 uses the operating controls to advance the distal end of catheter 28 to a brain location 60, seen on a display 56, where blood vessel sharply bends. The processor presents results of the catheter tracking procedure on display 56.

Processor 40 uses software stored in a memory 42 to operate system 20a. The software may be downloaded to processor 40 in electronic form, over a network, for example, or it may, alternatively or additionally, be provided and/or stored on non-transitory tangible media, such as magnetic, optical, or electronic memory. In particular, processor 40 runs a dedicated algorithm that enables processor 40 to perform the disclosed steps, as described below.

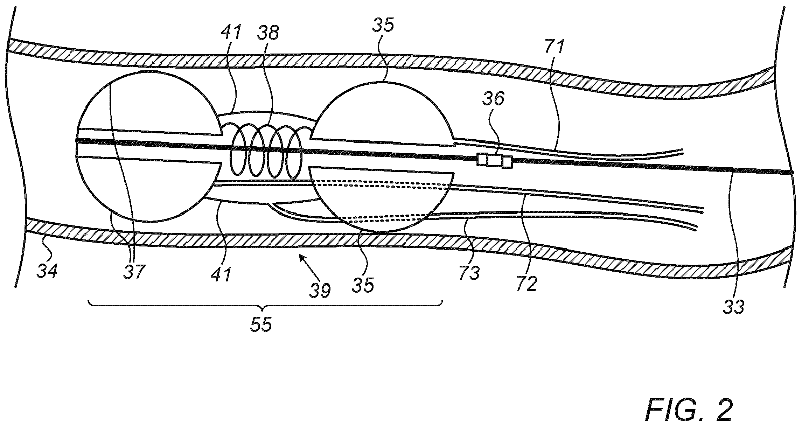

As seen on display 56, the distal end of catheter 28 comprises a guidewire advancement mechanism (GAM) 55 attached to guidewire 33. Inset 45 shows guidewire 33, surrounded by a blood vessel wall 34, with GAM 55 connected at the distal end, where GAM 55 comprises a proximal balloon 35, a distal balloon 37, and a middle inflatable balloon 39 that couples each of the balloons one to the other. GAM 55 is described in more detail in Fig. 2.

In some embodiments, a triple-balloon inflation/deflation (I/D) unit 70 is included in console 50. Typically, (I/D) unit 70 comprises one or more pumps (not shown) that pump saline solution into and out of the balloons to inflate and deflate the balloons, respectively. Unit 70 further comprises a controller that is configured to control the one or more pumps to sequentially inflate and deflate the three balloons of GAM 55, including evacuating middle balloon 39 of saline solution to advance guidewire 33 in a step-wise fashion through bent lumens, as described below. During the "crawling" guidewire advancement mode, I/D unit 70 sequentially inflates and deflates proximal balloon 35 via a tube 71, distal balloon 37 via a tube 72, and middle balloon 39 via a tube 73.

System 20b, shown in Fig. 1B, has a different magnetic location pad design, namely a location pad 24b. As seen, location pad 24b is fixed to the bed, and irradiators 26b surround a patient headrest horizontally. In this example, system 20b lacks reference sensor 21, and therefore the head of the patient must be harnessed to keep it motionless. Other components of system 20b are generally identical to those of system 20a. A location tracking system using a location pad similar to location pad 24b and a magnetic position sensor disposed on a distal end of a hollow guidewire is described in U.S. Patent Application 16/234,601, filed December 28, 2018, entitled "Ear-Nose-Throat (ENT) Hollow Guidewire with Balloon," which is assigned to the assignee of the present patent application and whose disclosure is incorporated herein by reference.

Systems 20a and 20b shown in Figs. 1A and 1B are chosen purely for the sake of conceptual clarity. Other system elements may be included, for example additional controls on handle 29 for controlling the diagnostic tooling designed to determine clot type. CARTO® magnetic tracking systems, which track a location and orientation of a magnetic position sensor in an organ of a body using techniques similar to those applied by systems 20a and 20b, are produced by Biosense-Webster.

### ADVANCING A GUIDEWIRE IN A BRAIN LUMEN

Fig. 2 is a schematic cross-sectional view of a tortuous section of brain vessel 34 and of a guidewire advancement mechanism (GAM) 55 configured to transit the tortuous section, in accordance with an embodiment of the present invention. Physician 54, who advances guidewire 33 of catheter 28 distally in brain vessel 34, encounters difficulty in advancing guidewire 33 and applies GAM 55 to further distally advance guidewire 33.

As seen, GAM 55, of a typical size of up to few millimeters in diameter and of several millimeters in length, is coupled to a distal end of guidewire 33, where GAM 55 comprises proximal balloon 35, distal balloon 37, and middle inflatable balloon 39 that that is coupled between the proximal and distal balloons. In the present example, the end of guidewire 33 is fixed to distal balloon 37. Middle inflatable balloon 39 comprises a variable-length element, e.g., a compressible/extendable element, such as spring or a telescopic rod, where Fig.2 shows, by way of example, a spring 38. The variable-length element may be made of plastics or metal, depending on the mode of operation. For example, a telescopic-rod can be made of plastic, whereas a spring is typically made of metal.

To enable sequential inflation and deflation of the balloons of GAM 55, proximal balloon 35 is connected to balloon inflation/deflation (I/D) unit 70 via tube 71, distal balloon 37 is connected to unit 70 via a tube 72, and middle balloon 39 is connected to unit 70 via a tube 73. Middle balloon 39 can be evacuated via tube 73 to be in sub-pressure.

In some embodiments, guidewire 33 of catheter 28 comprises a magnetic position sensor 36, which is used for tracking the position of guidewire 33 in the brain to assist physician 54 in navigating guidewire 33 in the brain as described in Figs. 1A and 1B.

GMA 55 is illustrated in Fig. 2 purely by way of example and in a simplified manner for the sake of conceptual clarity. For example, in another embodiment, spring 38 may be replaced with another elastic element and/or a compressible element that can similarly extend and contract, such as described above.

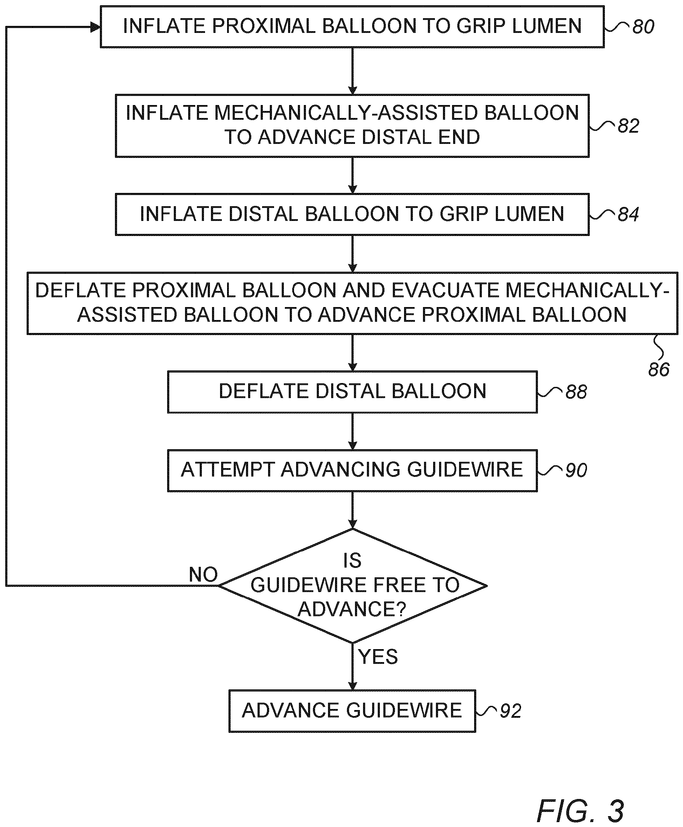

Fig. 3 is a flow chart that schematically illustrates a method for advancing a guidewire via a tortuous section of a brain vessel using GAM 55 of Fig. 2, in accordance with an embodiment of the present invention. The process begins with physician 54 encountering difficulty in advancing guidewire 33 beyond a certain location in blood vessel 34, and starts the "crawling" of GMA 55 distally to advance the guidewire, which is done when physician 54 inflates proximal balloon 35 to anchor the balloon 35 against the lumen (i.e., grip blood vessel 34), at a proximal balloon inflation step 80. Next, at a guidewire advancement step 82, physician 54 inflates middle balloon 39 to advance deflated distal balloon 37 distally, with balloon 39 being aided by the expansion force of spring 38.

Next, physician 54 inflates distal balloon 37 to anchor the balloon 37 against the lumen at the distally advanced position of balloon 37, at a distal balloon inflation step 84. Next, physician 54 deflates proximal balloon 35, and evacuates middle balloon 39 to let spring 38 contract and pull distally proximal balloon 35, at a proximal balloon advancement step 86. At this point, physician 54 deflates distal balloon 37, in a balloon deflation step 88, and attempts to freely advance guidewire 33, at a guidewire advancing attempt step 90. If guidewire 33 still cannot be further advanced distally by normal operation (e.g., pushing guidewire 33), physician 54 continues with the "crawling" of GMA 55 by returning to step 80. If guidewire 33 advances easily, then physician 54 continues to advance guidewire 33 normally, at a guidewire advancement step 92.

The example flow chart shown in Fig. 3 is chosen purely for the sake of conceptual clarity. In alternative embodiments, physician 54 may use the disclosed method to retract the guidewire, by I/D unit 70 inflating and deflating the balloons in another predefined order: (a) inflating the distal balloon, (b) inflating the middle balloon, (c) inflating the proximal balloon; and, (d) deflating the distal balloon and the middle balloon. Furthermore, the disclosed technique may include additional steps, such as rotating guidewire 33 about its longitudinal axis.

Although the embodiments described herein mainly address cerebrovascular applications, the methods and systems described herein can also be used in other organs, such as in biliary duct, urinary and ENT applications, and more.

It will be thus appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A medical probe, comprising:
a guidewire for insertion into a lumen of an organ of a patient; and
a guidewire advancement mechanism (GAM) disposed at a distal end of the guidewire, the GAM comprising:
a proximal inflatable balloon;
a distal inflatable balloon; and
a middle inflatable balloon that is coupled between the proximal and distal balloons, wherein the proximal, distal and middle balloons are configured to move the guidewire in the lumen by inflating and deflating in a predefined sequence.

2. The medical probe according to claim 1, and comprising a variable-length element, which is fitted inside the middle balloon and connects the distal balloon and the proximal balloon.

3. The medical probe according to claim 2, wherein the variable-length element comprises a spring.

4. A system, comprising:
a medical probe, comprising:
a guidewire for insertion into a lumen of an organ of a patient;
a guidewire advancement mechanism (GAM) disposed at a distal end of the guidewire, the GAM comprising:
a proximal inflatable balloon;
a distal inflatable balloon; and
a middle inflatable balloon that is coupled between the proximal and distal balloons, wherein the proximal, distal and middle balloons are configured to move the guidewire in the lumen by inflating and deflating in a predefined sequence;
and
an inflation/deflation (I/D) unit comprising a controller and one or more pumps configured to cause the GAM and the guidewire to move in the lumen, by inflating and deflating the proximal, distal and middle balloons in a predefined sequence.

5. The system according to claim 4, wherein the I/D unit is configured to advance the GAM and the guidewire in the lumen by applying the following predefined sequence:
inflating the proximal balloon;
inflating the middle balloon;
inflating the distal balloon; and
deflating the proximal balloon and the middle balloon.

6. The system according to claim 4, wherein the I/D unit is configured to retract the GAM and the guidewire in the lumen by applying the following predefined sequence:
inflating the distal balloon;
inflating the middle balloon;
inflating the proximal balloon; and
deflating the distal balloon and the middle balloon.

7. A method, comprising:
inserting, into a lumen of an organ of a patient, a guidewire whose distal end is connected to a guidewire advancement mechanism (GAM), the GAM comprising:
a proximal inflatable balloon;
a distal inflatable balloon; and
a middle inflatable balloon that is coupled between the proximal and distal balloons; and
moving the GAM and the guidewire in the lumen by inflating and deflating the proximal, distal and middle balloons of the GAM in a predefined sequence.

8. The method according to claim 7, wherein moving the GAM and the guidewire comprises advancing the GAM and the guidewire in the lumen by sequentially performing the steps of:
inflating the proximal balloon;
inflating the middle balloon;
inflating the distal balloon; and
deflating the proximal balloon and the middle balloon.

9. The method according to claim 7, wherein moving the GAM and the guidewire comprises retracting the GAM and the guidewire in the lumen by sequentially performing the steps of:
inflating the distal balloon;
inflating the middle balloon;
inflating the proximal balloon; and
deflating the distal balloon and the middle balloon.
